**Europäisches Patentamt**

**European Patent Office**

**Office européen des brevets**

(19)

(11) Veröffentlichungsnummer : **0 384 217 B1**

(12)

# EUROPÄISCHE PATENTSCHRIFT

(45) Veröffentlichungstag der Patentschrift :
**28.10.92 Patentblatt 92/44**

(51) Int. Cl.$^5$ : **G01N 1/24**

(21) Anmeldenummer : **90102371.3**

(22) Anmeldetag : **07.02.90**

(54) **Vorrichtung zur Förderung einer Messprobe in die Messkammer eines Messfühlers und Messverfahren dazu.**

(30) Priorität : **18.02.89 DE 3904994**

(43) Veröffentlichungstag der Anmeldung :
**29.08.90 Patentblatt 90/35**

(45) Bekanntmachung des Hinweises auf die
Patenterteilung :
**28.10.92 Patentblatt 92/44**

(84) Benannte Vertragsstaaten :
**DE FR IT NL**

(56) Entgegenhaltungen :
**EP-A- 0 153 883**
**EP-A- 0 302 681**
**DE-A- 2 035 982**
**DE-A- 2 944 444**
**GB-A- 2 184 245**

(73) Patentinhaber : **Drägerwerk**
**Aktiengesellschaft**
**Moislinger Allee 53-55**
**W-2400 Lübeck 1 (DE)**

(72) Erfinder : **Stock, Burkhard, Dr.**
**Sauerbruchweg 1**
**W-2400 Lübeck (DE)**

EP 0 384 217 B1

**Beschreibung**

Die Erfindung betrifft eine Vorrichtung zur Förderung einer gasförmigen Probe in die Meßkammer eines Nachweisgerätes zur Bestimmung des Bestandteiles einer Komponente eines Gases, insbesondere des Anteils von Alkohol in der Atemluft, mit Hilfe einer Zylinder-Kolben-Einheit als Förderelement, die die Probe aus einem Gasvorrat über einen Einlaß in die mit einem Fühler in Strömungsverbindung gebrachte Meßkammer befördert, von welcher ein Wandteil des als Meßkammer ausgebildeten Hubraums der Fördereinheit die meßempfindliche Oberfläche des Meßfühlers bildet. Außerdem wird ein Meßverfahren hierzu angegeben.

Eine derartige Vorrichtung aus der DE-OS 20 35 982 besitzt eine Kolben-Zylindereinheit, die aus einem Ausatemmundstück, welches als Gasvorrat dient, eine zu untersuchende Meßprobe im Nebenstrom absaugt, welche in dem Hubvolumen verwirbelt und in einem Ausblashub einem Analysator zugeführt wird. Bei dieser bekannten Ausführungsform ist es nachteilig, daß während des Ansaugens der Luftprobe aus dem Vorrat an den Wandungen des Hubvolumens in der Kolben-Zylinder-Einheit Gasmoleküle adsorbiert werden oder gar kondensieren und somit während des Ausblashubes nicht zu dem Nachweisgerät gelangen können. Außerdem verbleibt nach Beendigung des Ausblashubes eine Restmenge der Meßprobe im Hubvolumen bzw. den Zuführungsleitungen zum Nachweisgerät zurück, welche einer Auswertung unzugänglich sind und somit das Meßergebnis in Richtung zu geringer Nachweismengen verfälscht.

Darüber hinaus besitzen Kolben-Zylindereinheiten als Förderelemente den Nachteil, daß zwischen dem Kolben und dem Zylinder ein recht hoher Reibungskoeffizient vorliegt, welcher einen erheblichen Leistungsverlust während des Betriebes nach sich zieht. Dieser ist besonders unerwünscht für den Fall, daß tragbare batteriebetriebene Geräte eingesetzt werden sollen. Der sich ergebende relativ hohe Reibungskoeffizient wird im wesentlichen dadurch bestimmt, daß zwischen dem Kolben und dem Zylinder eine möglichst gute Abdichtung gefordert wird, um während des Pumpvorganges Leckagen oder angesaugte Fehlluft möglichst auszuschließen. Eine andere Vorrichtung nach der GB-A 2 044 462 besitzt ein Förderelement, durch dessen Betätigung die Meßprobe während des Ansaugevorganges an dem Meßfühler vorbei befördert wird. Hierbei ist es jedoch von Nachteil, daß während des Ansaugevorgangs nicht alle Moleküle genügend Zeit haben, mit dem Meßfühler zu reagieren, um einen Beitrag zum Nachweissignal zu liefern. Der Anteil der nicht mit dem Meßfühler reagierenden Probenmenge hängt von der Reaktionsgeschwindigkeit an der meßempfindlichen Oberfläche des Meßfühlers im Verhältnis zur Pumpgeschwindigkeit ab.

In der GB-A-2 184 245 ist ein Meßgerät beschrieben, bei dem die Meßkammer den Hubraum eines Kolbens darstellt, in welchen einerseits die zu untersuchende Gasprobe über einen Einlaß Zutritt hat, und von der andererseits der der Kolbenstirnfläche gegenüberliegende Wandteil als meßempfindliche Oberfläche ausgebildet ist. Zur Probenahme wird der verriegelte Kolben freigesetzt, so daß er unter Federkraftwirkung eine den Hubraum vergrößernde Wegstrecke zurücklegt, wodurch die zu untersuchende Probenmenge durch den Einlaß in die Meßkammer eintritt und diese füllt. Die zu messende Gaskomponente (Atemalkohol) durchdringt bei ruhendem Kolben die gesamte Meßkammer und gelangt, größtenteils durch Diffusion, zu der Meßoberfläche, welche die Elektrodenoberfläche einer elektrochemischen Meßzelle (Brennstoffzelle) darstellt.

Bei diesem bekannten Meßgerät gelangt die Probenmenge je nach Verwirbelungsgrad und Diffusionsfähigkeit in unbeeinflußbarer Weise, in die gesamte Meßkammer. Es können sich strömungsmäßig unberücksichtigte Toträume auf der Meßoberfläche bilden, die von einer Gasprobe nicht ausgespült und daher nicht von der zu untersuchenden Gaskomponente erfaßt werden.

Der vorliegenden Erfindung liegt somit die Aufgabe zugrunde, eine Vorrichtung der genannten Art so zu verbessern, daß eine möglichst vollständige Ausnutzung der Meßprobe zur Signalauswertung durch den Meßfühler verwirklicht wird und dies ohne hohe Verlustleistungen durchgeführt werden kann.

Die Lösung der Aufgabe erfolgt dadurch, daß im Strömungsverlauf der Meßprobenführung zwischen dem Einlaß und dem Hubraum eine Umwegführung entlang der meßempfindlichen Oberfläche des Meßfühlers vorgesehen ist.

Der Vorteil der Erfindung liegt im wesentlichen darin, daß eine intensivere Wechselwirkung zwischen der gasförmigen Meßprobe und dem Meßfühler erzielt wird. Die Meßprobenmoleküle besitzen während des Pumpvorganges eine genügend lange Verweilzeit an der meßempfindlichen Oberfläche, so daß der größte Anteil der nachzuweisenden Komponente schon mit dem Meßfühler reagiert hat, bevor er in den Hubraum gelangt. Nach abgeschlossenem Ansaughub des Förderelementes verbleiben somit nur mehr eine geringe Anzahl der Nachweismoleküle im Hubraum, die während des anschließenden Stillstandes des Kolbens zu der Meßoberfläche diffundieren und einen Beitrag zum Meßsignal liefern. Auf diese Weise erhält man eine vollständige Ausnutzung der Meßprobe bei einer hohen Ansprechgeschwindigkeit durch das Entlangführen der Meßprobe an der Meßoberfläche, weil größere Diffusionswege im Pumpenraum nur noch für einen kleinen Bruchteil der Moleküle im Hubvolumen von Bedeutung sind. Besonders vorteilhaft wirkt sich diese Umwegführung dann aus, wenn ein elektrochemischer Sensor zum Nachweis benutzt wird, der beispielsweise Schwefelsäure als Elektrolyt enthält. Dann wird nämlich der bei der Ausatmung in der Meßprobe enthaltene Wasserdampf während des Vorrüberstreichens an der Sensoroberfläche aufgenommen (Trocknung der Meßprobe), so daß eine Kondensation bei niedrigen Temperaturen im Pumpenraum vermieden wird. Eine besonders einfache Ausführungsform der Umwegführung wird dadurch verwirklicht, daß der Einlaß in eine Vorkammer zum Hubraum mündet, welche einerseits die Meßfläche des Fühlers und andererseits die nichtbewegliche, mit einem Durchbruch versehene Stirnwand des Hubraumes als Wandteile besitzt, wobei ein den Durchbruch teilweise umgebender, entlang des Umfangs der Meßfläche verlaufender Bord die Umwegführung bildet. Nach abgeschlossener Ansaugung der Meßprobe und Verharren des Kolbens in seiner Endstellung des Ansaughubs können die verbliebenen nachzuweisenden Moleküle aus dem Hubraum über den Durchbruch zur Meßoberfläche direkt diffundieren, so daß eine vollständige Ausnutzung der Meßprobe z. B. für eine amperometrische Auswertung zur Verfügung steht. Durch die kurzen Diffusionswege bleibt die Ansprechgeschwindigkeit hoch und die Meßdauer kurz.

Um eine verbesserte Abdichtung von Kolben und Zylinder bei möglichst geringer Haftreibung zu erzielen, ist es zweckmäßig, den aus graphitiertem Kohlenstaub gepreßten Kolben in einem Glaszylinder laufen zu lassen. Dadurch wird der Vorteil erzielt, daß durch die Schmiereigenschaft des Graphits ein sehr geringer Reibungskoeffizient auch bei genauestmöglicher Anpassung der Glas-Graphit-Paarung erzielt werden kann, so daß eine geringe Reibung bei hoher Dichtigkeit vorhanden ist. Damit wird ein hoher Wirkungsgrad der Pumpe erzielt, der bei batteriebetriebenen Geräten notwendig ist. Darüber hinaus wird eine hohe Lebensdauer durch geringsten Abrieb und eine gute Abkapselung des Meßfühlers von der Außenluft auch bei ruhendem Kolben erreicht. Da Glas und Graphit den gleichen Ausdehnungskoeffizienten besitzen, wird auch bei schwankenden Umgebungs- bzw. Betriebstemperaturen keine Temperaturstabilisierung des Förderelementes benötigt. Glas und Graphit sind chemisch inert, so daß Korrosionsgefahr durch die Bestandteile in der Meßprobe oder durch im Meßfühler möglicherweise enthaltenen Chemikalien, wie z. B. Schwefelsäure bei Einsatz eines elektrochemischen Sensors, keine nachteiligen Auswirkungen haben. Eine gleichgute Abdichtung mit ausreichender Paßgenauigkeit erreicht man durch eine Kolben-Zylinder-Paarung aus Glas, wobei die Laufflächen aufeinander eingeschloffen sind.

Ein Verfahren zur Messung eines Bestandteils in einer gasförmigen Probe, insbesondere zur Bestimmung des Alkoholgehaltes in der Atemluft, mit einer beschriebenen Vorrichtung besteht darin, daß der Kolben einen Ansaughub ausführt, durch den das Probengas durch den Einlaß entlang der Umwegführung über die Meßfläche geleitet und in den Hubraum gesaugt wird, worin es während einer festlegbaren Zeitdauer verweilt. Mit diesem Verfahren wird die Meßzeit verkürzt, indem mit einer hohen Ansprechgeschwindigkeit der größte Anteil der Meßprobe während des kurzen Ansaughubes schon auswertbar ist, z. B. dadurch, daß die Anstiegsgeschwindigkeit des vom Meßfühler aufgenommenen Meßsignals gemessen wird, und daß nach abgeschlossenem Ansaughub nur noch eine geringe Meßprobenmenge übrigbleibt, welche zum Zweck einer integrierenden Mengenbestimmung (amperometrisches Meßverfahren) in einer relativ kurzen Diffusionszeit von dem Meßfühler erfaßt werden kann.

Nach erfolgter Auswertung während der Verweilzeit führt der Kolben einen Ausblashub aus, um das ausgewertete Probengas aus dem Einlaß wieder auszutreiben. Dieser Ausblashub führt nicht mehr zu einem das Meßergebnis verfälschenden Signals am Meßfühler, weil nunmehr sämtliche zur Verfügung gestandenen Moleküle der vorher angesaugten Meßprobe durch die Reaktion mit dem Meßfühler aufgezehrt wurden, so daß das Probengas frei von Nachweismolekülen ist und gewissermaßen als inertes Abgas auf einfache Weise durch den Einlaß wieder ausgeblasen werden kann. Dadurch werden aufwendige und mechanisch anfällige Richtungsventilsteuerungen vermieden.

Ein Ausführungsbeispiel der Erfindung wird anhand der schematischen Zeichnung dargestellt und im folgenden näher erläutert.

Es zeigen

Figur 1 ein Nachweisgerät mit einem Meßfühler und Kolben-Pumpen-Aggregat,

Figur 2 die Draufsicht auf die Vorkammer bei abgenommenem Meßfühler.

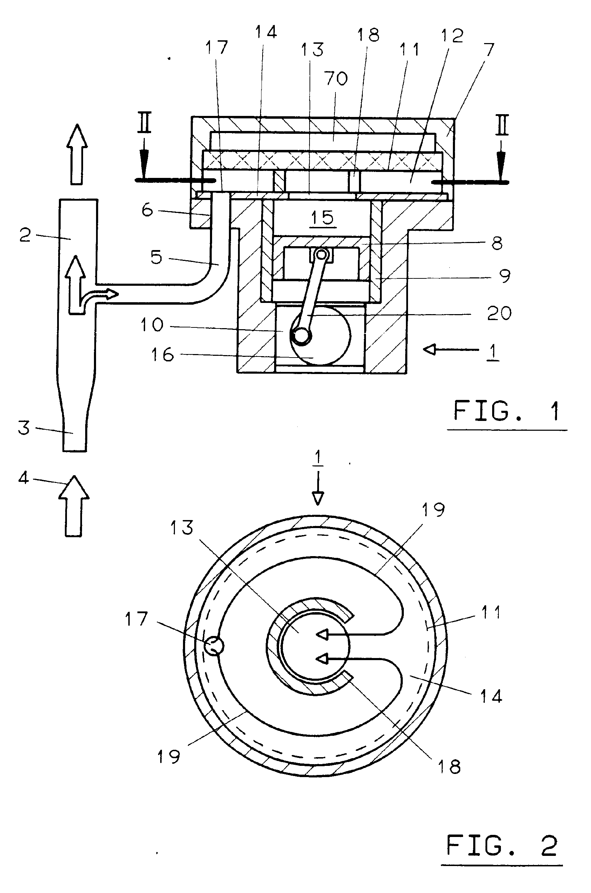

In Figur 1 ist ein Nachweisgerät (1) zur Bestimmung des Bestandteiles einer Komponente eines Gases, insbesondere des Anteils von Alkohol in der Atemluft, mit einer Probensammelvorrichtung (2) dargestellt. Das Ausführungsbeispiel soll anhand der Prüfung des Anteils von Alkohol in der Atemluft erläutert werden. Die Probensammelvorrichtung (2) besitzt ein Mundstück (3), welches ein nicht dargestellter Proband in den Mund nimmt und seine Ausatemluft in Richtung der Pfeile (4) durch die Probensammelvorrichtung (2) hindurchbläst. Radial von der Probensammelvorrichtung (2) zweigt eine Probenleitung (5) aus einem beheizten Kunststoffschlauch in das Gehäuse (6) des Nachweisgerätes (1) ab. In dem Nachweisgerät (1) sind im wesentlichen ein Meßfühler (7), eine Kolben-Zylindereinheit (8, 9) sowie ein Motorantrieb (10) enthalten. Der Meßfühler (7) besitzt eine Meßfläche (11), welche eine Vorkammer (12) von dem einen Elektrolyten enthaltenden Innenraum (70) des Meßfühlers (7) abtrennt. Die Vorkammer (12) ist als Erweiterung des Hubraumes (15) ausgebildet. Typischerweise besteht die Meßfläche (11) aus einer für den Alkoholdampf permeablen Membran. Zu der Kolben-Zylindereinheit (8, 9) ist die Vorkammer (12) von der einen Durchbruch (13) aufweisenden Stirnwand (14) zu dem Hubraum (15) hin abgeteilt. Der Hubraum (15) wird durch die Hubbewegung des Kolbens (8) verändert. In der dargestellten Form befindet sich der Kolben kurz vor dem oberen Totpunkt, der durch den Exzenter (16) an dem Motorantrieb (10) festgelegt ist. Der Kolben (8) wird mittels der Pleuelstange (20) im Zylinder (9) hin und her bewegt. Zum Ansaugen der Meßprobe wird der Kolben (8) durch den Motor (10) in seinen unteren Totpunkt verlagert und verharrt dort für einen gewissen Zeitraum. Während dieses Ansaughubes des Kolbens (8) wird aus dem durch die Probenahmevorrichtung (2) strömenden Atemluft eine Meßprobe durch die Probenleitung (5) über einen Einlaß (17) in die Vorkammer (12) angesaugt und von dort um einen eine Umwegführung bildenden Bord (18) durch den Durchbruch (13) in den Hubraum (15) gefördert. Während des Ansaughubs streicht die Meßprobe in der Vorkammer (12) entlang der Meßfläche (11) des Meßfühlers (7), und zwar möglichst weitläufig an dessen gesamter Flächenausdehnung entlang. Während dieser Zeit findet die wesentliche Umsetzung des Atemalkohols in ein Meßsignal statt. Der verbleibende, noch nicht umgesetzte Rest wird während der kurzen Verweilzeit im Hubraum (15) durch Diffusion an die Meßoberfläche (11) zur Auswertung herangezogen. Auf diese Weise erhält man eine möglichst umgehende Umsetzung der Meßprobe in ein Meßsignal und damit eine kurze Ansprechzeit des Nachweisgerätes (1), wobei auch zu einer amperometrischen Messung die vollständige Umsetzung des Nachweisgases in ein Meßsignal in einer recht kurzen Zeit erreicht werden kann.

In Figur 2 ist eine Draufsicht auf das Gehäuse (1) dargestellt, wobei der den Meßfühler (7) enthaltende Teil oberhalb des Schnittes II - II abgenommen ist. Die Draufsicht erfolgt somit in die geöffnete Vorkammer (12) und auf die den Bord (18) aufnehmende durchbrochene Stirnwand (14) der Fördereinheit (8, 9). Durch den Durchbruch (13) ist ein Teilbereich der Stirnfläche des Kolbens (8) erkennbar. Der Bord (18) umkreist den Durchbruch (13) an seinem Randumfang läßt jedoch ein dem Einlaß (17) gegenüberliegenden Bereich offen. Die Kontur der Meßfläche (11) ist gestrichelt auf der Stirnwand (14) eingezeichnet. Sie deutet an, daß bei aufgesetztem Meßfühler die zugehörige Meßfläche (11) in diesem Bereich aufliegt. Bei der Strömung der Meßprobe aus dem Einlaß (17) in den Zwischenraum (12) werden die Gasmoleküle entlang der Wegführung (19) in das Hubvolumen (15) angesaugt. Dabei überstreichen sie zwangsläufig einen Großteil der Meßfläche (11).

## Patentansprüche

1. Vorrichtung zur Förderung einer gasförmigen Probe in die Meßkammer (15) eines Nachweisgerätes zur Bestimmung des Bestandteiles einer Komponente eines Gases, insbesondere des Anteils von Alkohol in der Atemluft, mit Hilfe einer Kolben-Zylindereinheit (8, 9) als Förderelement, welche die Probe aus einem Gasvorrat über einen Einlaß (17) in die mit einem Meßfühler (7) in Strömungsverbindung gebrachte Meßkammer (15) befördert, von welcher ein Wandteil (11) des als Meßkammer ausgebildeten Hubraumes (15) der Fördereinheit (8, 9, 10) die meßempfindliche Oberfläche des Meßfühlers (7) darstellt, dadurch gekennzeichnet, daß im Strömungsverlauf der Meßprobenführung (19) zwischen dem Einlaß (17) und dem Hubraum (15) eine Umwegführung (18) entlang der meßempfindlichen Oberfläche (11) des Meßfühlers (7) vorgesehen ist.

2. Vorrichtung nach Anspruch 1, dadurch gekennzeichnet, daß der Einlaß (17) in eine Vorkammer (12) zum Hubraum (15) mündet, die einerseits die Meßfläche (11) des Meßfühlers (7) und andererseits die nicht bewegliche, mit einem Durchbruch (13) versehene Stirnwand (14) des Hubraums (15) als Wandteile besitzt, wobei ein den Durchbruch (13) teilweise umgebende, entlang des Umfangs der Meßfläche (11) verlaufende Bordumfassung (18) die Umwegführung bildet.

3. Vorrichtung nach Anspruch 1 oder 2, dadurch gekennzeichnet, daß der aus graphitiertem Kohlenstaub gepreßte Kolben (8) in einem Glaszylinder (9) läuft.

4. Vorrichtung nach Anspruch 1 oder 2, dadurch gekennzeichnet, daß der aus Glas gefertigte Kolben (8) in einem eingeschliffenen Glaszylinder (9) läuft.

5. Verfahren zur Messung eines Bestandteils in einer gasförmigen Probe, insbesondere zur Bestimmung des Alkoholgehaltes in der Atemluft, mit einer Vorrichtung nach einem der Ansprüche 1 bis 4, dadurch gekennzeichnet, daß der Kolben (8) einen Ansaughub ausführt, durch den das Probengas durch den Einlaß (17) entlang der Umwegführung (18) über die Meßfläche (11) geleitet und in den Hubraum (15) gesaugt wird, worin es während einer festlegbaren Zeitdauer verweilt.

6. Verfahren nach Anspruch 5, dadurch gekennzeichnet, daß nach der Verweilzeit der Kolben (8) einen Ausblashub ausführt und das ausgewertete Probengas aus dem Einlaß (17) austreibt.

## Claims

1. Device for conveying a gaseous sample into the measuring chamber (15) of a detecting device for the purpose of determining the constituent of a component of a gas, in particular the proportion of alcohol in the respiratory air, with the aid of a piston-cylinder unit (8, 9) as a conveying element which conveys the sample from a gas supply by way of an inlet (17) into the measuring chamber (15) which is connected in terms of flow with a measuring sensor (7) and of which a wall portion (11) of the stroke area (15) of the conveying unit (8, 9, 10) formed as the measuring chamber represents the measurement-sensitive surface of the measuring sensor (7), characterised in that provided in the path of flow of the measurement sample guide (19) between the inlet (17) and the stroke area (15) there is a by-pass guide (18) along the measurement-sensitive surface (11) of the measuring sensor (7).

2. Device according to claim 1, characterised in that the inlet (17) runs into a pre-chamber (12) with respect to the stroke area (15), which chamber has as wall portions, on the one side, the measuring surface (11) of the measuring sensor (7) and, on the other side, the end wall (14) of the stroke area (15) which is not movable and which is provided with an opening (13), with a border enclosure (18), which partly surrounds the opening (13) and extends along the periphery of the measuring surface (11), forming the by-pass guide.

3. Device according to claim 1 or 2, characterised in that the piston (8) which is pressed out of graphite-coated coal dust runs in a glass cylinder (9).

4. Device according to claim 1 or 2, characterised in that the piston (8) which is made of glass runs in a ground-in glass cylinder (9).

5. Method for measuring a constituent in a gaseous sample, in particular for determining the alcohol content in the respiratory air, having a device according to one of the claims 1 to 4, characterised in that the piston (8) executes an intake suction stroke, by means of which the sample gas is directed through the inlet (17) along the by-pass guide (18) over the measuring surface (11) and is sucked into the stroke area (15), in which it dwells for a period of time which can be fixed.

6. Method according to claim 5, characterised in that after the dwell time the piston (8) executes a blow-out stroke and drives the evaluated sample gas out of the inlet (17).

## Revendications

1. Dispositif pour le transport d'un échantillon gazeux dans la chambre de mesure (15) d'un appareil de détection en vue de déterminer la proportion d'un composant du gaz, en particulier la proportion d'alcool contenue dans l'haleine, à l'aide d'un ensemble cylindre - piston (8, 9) servant d'élément de transport, qui envoie l'échantillon d'une réserve de gaz, par une entrée (17), dans la chambre de mesure (15) communiquant avec une sonde de mesure (7), chambre à partir de laquelle une partie de paroi (11) de la cylindrée (15), conçue comme chambre de mesure, de l'unité de transport (8, 9, 10), constitue la surface sensible à la mesure de la sonde de mesure (7), caractérisé en ce qu'il est prévu, dans le parcours d'écoulement du conduit de l'échantillon à mesurer (19), entre l'entrée (17) et la cylindrée (15), une déviation (18), le long de la surface (11) sensible à la mesure de la sonde (7).

2. Dispositif selon la revendication 1, caractérisé en ce que l'entrée (17) débouche dans une avant-chambre (12) menant à la cylindrée (15), qui présente, en tant que parties de paroi, d'une part, la surface de mesure (11) de la sonde de mesure (7) et d'autre part, la paroi frontale (14) non mobile, pourvue d'un ajour (13), de la cylindrée (15), une monture (18) entourant partiellement l'ajour (13), s'étendant le long du pourtour de la surface de mesure (11), formant la déviation.

3. Dispositif selon les revendications 1 ou 2, caractérisé en ce que le piston (8) en poudre de carbone graphité, pressé, coulisse dans un cylindre en verre (9).

4. Dispositif selon les revendications 1 ou 2, caractérisé en ce que le piston (8) en verre coulisse dans un cylindre en verre (9) émerisé.

5. Procédé de mesure d'un constituant d'un échantillon gazeux, en particulier en vue de déterminer la teneur en alcool de l'haleine, au moyen d'un dispositif selon l'une des revendications 1 à 4, caractérisé en ce que le piston (8) exécute une course d'aspiration par laquelle le gaz de l'échantillon est envoyé, par l'entrée (17), le long de la déviation (18), sur la surface de mesure (11) et est aspiré dans la cylindrée (15) où il séjourne pendant une durée à déterminer.

6. Procédé selon la revendication 5, caractérisé en ce qu'après la durée de séjour, le piston (8) exécute une course de refoulement et chasse le gaz de l'échantillon analysé, par l'entrée (17).

FIG. 1

FIG. 2